# EUROPEAN PATENT APPLICATION

(11) **EP 2 514 737 A1**
(43) Date of publication of application: **24.10.2012**
(21) Application number: 10837007.3
(22) Date of filing: 29.11.2010
(51) Int. Cl.: C07C 41/34, C07C 29/76, C07C 43/04, C07C 31/125, B01D 11/04, B01D 3/14

(54) **PROCESS AND APPARATUS FOR COUPLING SEPARATION AND PURIFICATION OF ETHYL TERT-BUTYL ETHER**

(30) Priority: 16.12.2009 CN 200910201215
(71) Applicant: East China University Of Science And Technology, Shanghai 200237 (CN)
(72) Inventor: ZHANG, Wuping, Shanghai 200237 (CN); ZHOU, Shanhua, Shanghai 200237 (CN); ZHANG, Wenli, Shanghai 200237 (CN); TIAN, Hengshui, Shanghai 200237 (CN); ZHANG, Hanlu, Shanghai 200237 (CN); ZHU, Fugeng, Shanghai 200237 (CN); GU, Jingjun, Shanghai 200237 (CN)
(74) Representative: Brown, David Leslie
(86) International application number: PCT/CN2010/079214
(87) International publication number: WO 2011/072570

(57) **Abstract**

A process and device of coupling separation and purification to produce ethyl tert-butyl ether (ETBE) is provided, wherein ethanol containing 1 wt% to 15 wt% of water, and a mixture containing ETBE and ethanol are fed into the distillation-extraction coupling zone for separation and purification. Said distillation-extraction coupling zone is composed of a distillation column and an extraction column. Said process and device can achieve the cogeneration of ETBE and ethanol, and possesses high commercial values.

## Description

### FIELD OF THE INVENTION

The present invention relates to a process and device to produce ethyl tert-butyl ether (ETBE), more particularly to a process and device of purification to produce ETBE. The purification process to produce ETBE is carried out by coupling separation.

### BACKGROUND OF THE INVENTION

The growth of biofuel of using ethanol (EtOH) and the like produced by fermentation as the base has attracted more and more attention in many countries.

However, one of the key issues that constrains the energy consumption and cost to produce fuel alcohol which is currently the most important bio-based alcohol ether fuel is that, if it is intended to reduce approximately 5wt% of azeotropic water to below 1 wt%, special separation methods have to be adopted such as azeotropic distillation, extractive distillation, adsorption and pervaporation membrane separation. Meanwhile, a small amount of denaturants (e.g. fusel alcohol, ethers, etc.) enhancing cosolubilization or solubilization has to be added to offset to some extent the disadvantage of phase separation that alcogas is likely to occur. In spite of the above fact, the price of ethanol fuel is relatively lower than gasoline because of its own inadequate capacities, and in some countries, such as China, its production cost is even higher than gasoline, in other words, the value (i.e. functions/costs) of ethanol fuel is on the low side; therefore it is necessary to find a solution.

It is already known that ETBE can be used as gasoline additive.

Generally, ETBE, used as a gasoline additive, is produced by means of etherification of isobutene contained in the fraction of ethanol and mixed C₄-hydrocarbons (e.g. from steam cracking or catalytic cracking) (see US 5248836A, CN 1990443A, CN1772848A and CN101195560A). It is easy to make ETBE, but many problems need to be solved when ETBE has to be separated and purified before being used as the gasoline additive.

In order to improve product functions and convenience of use, especially when ETBE can be directly added to the gasoline during blending without having to change the existing transport and distribution system, it is essential to control the ethanol content during the preparation of ETBE, when necessary, the ethanol content can be controlled below 1 wt%.

Until now, with respect to the process of separation and purification to produce ETBE, in some processes, the ethanol content entering into the debutanizer is strictly controlled, including the process to control ethanol to olefine ratio below a certain stoichiometric ratio and to conduct pretreatment (such as removing part of ethanol with water and adding a certain amount of hydrocarbons) before ethanol enters into the debutanizer (see US 7141705A, US 5447607A and US 5536886A). In such a way, the rest of ethanol can almost completely be removed with relevant hydrocarbons from the top of the debutanizer by azeotropy. But it will become difficult to further increase the conversion of isoolefme under some circumstances, including reactive distillation which is often desired to practise; therefore it may be required to add a certain amount of ethanol at the top of the debutanizer. Consequently, it may be difficult or restricted to increase the conversion of isoolefine when the ethanol content entering into the debutanizer is strictly controlled.

On the contrary, it is difficult to control the unreacted ethanol content entering into the debutanizer if it is intended to increase the conversion of isoolefine. There are two main categories of process according to different locations of the debutanizer to take off materials:

One is that the material is taken off the bottom of the debutanizer followed by further separation (see US 5,158,652A, US 5250156A, US 5,348,624A and US 5569787A); and

The other one is that the material is taken off at the side of the debutanizer followed by further separation (see CN1127243Aand US 5,607,557A).

Though each process above-mentioned has its merits, they share the common deficiency: strict requirements on the content of some components entering into the debutanizer are posed, for example, the water content of the material stream entering into the debutanizer is required to be 1 wt% maximal, the tert-butyl alcohol content is at the most 1 wt% (see CN1127243A and US 5,607,557A), and/or special separation methods have to be taken such as adsorptive separation, pervaporation membrane separation and azeotropic separation. Accordingly, the commercial value of the process of separation and purification to produce ETBE is decreased due to the strict operating conditions and/or the separation methods with higher energy consumption.

### SUMMARY OF THE INVENTION

In view of the above-described problems, it is one intention of the present invention to provide a process and device of purification to produce ETBE with commercial value (i.e. functions/costs). The purification process to produce ETBE is carried out by coupling separation. Said ETBE comes from a mixture of ETBE and ethanol, specifically, it is from the mixture of ETBE and ethanol obtained through the reaction of ethanol and mixed C₄-hydrocarbons (e.g. from steam cracking or catalytic cracking) containing isobutylene. The ETBE obtained by means of purification may contain no more than 1 wt% of ethanol. In other words, the obtained ETBE products have high capabilities as a gasoline additive.

Another objective of the invention is to provide a process and device of purification to produce ETBE with commercial value (i.e. functions/costs), using ethanol containing at least higher than 1 wt% up to 15 wt% of water, as the feedstock. Said purification process to produce ETBE is carried out by coupling separation. Said ethanol obtained by means of purification to produce ETBE may contain no more than 1 wt% of water, or even no more than 0.5 wt% of water. It can be used as the feed for etherification and/or used as a gasoline additive as required. In other words, the obtained ethyl tert-alkyl ether (ETAE) products can pocess high capabilities when they are used as gasoline additives, and meanwhile the costs are significantly reduced and the co-produced ethanol almost contains no azeotropic water.

Said ETBE is obtained by separation and purification of a mixture mainly containing ETBE and ethanol, specifically, it is obtained by separation and purification of a mixture mainly containing ETBE and ethanol obtained through etherification of ethanol and mixed C₄-hydrocarbons (e.g. originated from steam cracking or catalytic cracking) containing isobutylene, referring to US 5248836A, CN 1990443A, CN1772848A and CN101195560A for detailed information. Generally, the mixture mainly containing ETBE and ethanol obtained at the bottom or side of the debutanizer behind the reaction zone contains 5%-50 wt% of ethanol or more commonly 10%-40 wt% of ethanol, including the circumstance using the total weight of ETBE and ethanol obtained from the side of the debutanizer as the reference (see CN1127243A and US 5,607,557A for details). Apart from that, the mixture mainly containing ETBE and ethanol may further contains a small amount of other substances such as tertiary butyl alcohol (TBA), diisobutylene, ethyl sec-butyl ether (E2BE), diethyl ether and C₅-hydrocarbons, during the separation and purification of ETBE and ethanol, those substances can be seen identical with ETBE without subdivision (except that tertiary butyl alcohol is left in the ethanol after separation and purification since they have the similar boiling point).

The process of purification to produce ETBE mainly includes the following steps: feeding the mixture (obtained at the bottom or side of the debutanizer behind the reaction zone used to produce ETBE) mainly containing ETBE and ethanol as well as the ethanol containing higher than 1 wt% up to 15 wt% of water into a distillation-extraction coupling zone for coupling separation and purification, wherein the distillation-extraction coupling zone is composed of a distillation column and an extraction column. As a result, not only the in spec ETBE products can be obtained from the top of the extraction column but the ethanol almost having no azeotropic water can also be co-produced at the bottom of the distillation column.

The technical solution of the present invention is as follows:

A process of coupling separation and purification to produce ETBE comprises the following steps:

Introduce the ethanol containing higher than 1 wt% up to 15 wt% of water, preferably, the make-up ethanol containing higher than 1 wt% up to 15 wt% of water and/or the recovered ethanol or the mixture thereof as well as the mixture mainly containing ETBE and ethanol into a distillation-extraction coupling zone, wherein the distillation-extraction coupling zone is composed of a distillation column and an extraction column.

In said distillation-extraction coupling zone, the fraction containing ethanol is collected at the bottom of the distillation column, the material collected at the top of the distillation column is fed into the extraction column and extracted by water as the extraction agent, the extract liquor containing ethanol is collected at the bottom of the extraction column and reused (e.g. reintroduced into the distillation-extraction coupling zone) after the ethanol is recovered, while the extraction raffinate containing ETBE is collected at the top of the extraction column.

A preferred technical solution of the present invention, wherein said ETBE comes from the mixture (obtained at the bottom or side of the debutanizer behind the reaction zone used to produce ETBE) mainly containing ETBE and ethanol, comprises the following steps:

Introduce the ethanol containing higher than 1 wt% up to 15 wt% of water, preferably, the make-up ethanol containing 1.5%-15 wt% of water, more preferably, the make-up ethanol containing 2%-10 wt% of water, most preferably, the make-up ethanol containing 3%-10 wt% of water and/or the recovered ethanol or the mixture thereof as well as the mixture mainly containing ETBE and ethanol into a distillation-extraction coupling zone, wherein the distillation-extraction coupling zone is composed of a distillation column and an extraction column.

In said distillation-extraction coupling zone, the fraction containing ethanol is collected at the bottom of the distillation column, the material collected at the top of the distillation column is fed into the extraction column and extracted by water as the extraction agent, the extract liquor containing ethanol is collected at the bottom of the extraction column and reused (e.g. reintroduced into the distillation-extraction coupling zone) after the ethanol is recovered, while the extraction raffinate containing ETBE is collected at the top of the extraction column.

The technical solution of the device provided by the invention is as follows:

A device of coupling separation and purification to produce ETBE comprises a distillation column **C1** and an extraction column **C2.**

The distillation column **C1** is disposed with a connecting pipeline **3** to introduce distillation feed. The bottom of the distillation column is disposed with a heater **E3** and a pipeline **4** to collect the fraction containing ethanol. The top of the distillation column is disposed with a condenser **E2** and a condensate tank **B1** to collect fractions containing ETBE, ethanol and water. The top of the distillation column is also disposed with a pipeline **5** to feed the material obtained from the top of the distillation column **C1** to the extraction column **C2.** The distillation column further comprises a pipeline **6** used to feed extraction agent into the extraction column **C2,** a pipeline **7** used to collect the extract liquor containing ethanol at the bottom of the extraction column **C2** and a pipeline **8** used to collect the extraction raffinate containing ETBE at the top of the extraction column **C2.**

In accordance with the process and device of purification to produce ETBE of the invention, the purification process to produce ETBE is carried out by coupling separation. The ETBE obtained by means of purification may contain no more than 1 wt% of ethanol and the obtained ethanol may contain no more than 1 wt% of water. According to the process and device provided by the invention, no strict restriction, such as ethanol content and/or water content and/tertiary alcohols content is posed to the mixture (obtained at the bottom or side of the debutanizer behind the reaction zone) mainly containing ETBE and ethanol. The renewable ethanol containing at least higher than 1 wt% up to 15 wt% of water and the mixture to be separated mainly containing ETBE and ethanol that are hard to be directly separated can be coupled together for coupling separation in the distillation-extraction coupling zone to achieve multiple coupling such as cogeneration coupling of ETBE and ethanol products. Meanwhile, ethanol feed can be provided for etherification and/or ethanol products can be provided as gasoline additive as required. The ethanol/olefine ratio can be appropriately increased or loosen for the etherification so as to increase the conversion of isobutene and/or the selectivity of etherification within a certain range. Therefore, the commercial value (i.e. functions/costs) of the invention is very high.

### BRIEF DESCRIPTION OF THE DRAWINGS

FIG. 1 shows a flow diagram of a process and device of coupling separation and purification to produce ETBE of the invention;
FIG. 2 shows a flow diagram of the process (including the process to remove the material from the bottom of debutanizer area) of coupling separation and purification to produce ETBE of the invention; and
FIG. 3 shows a flow diagram of the process (including the process to remove the material from the side of debutanizer area) of coupling separation and purification to produce ETBE of the invention;

Wherein,
**C1:** distillation column;
**C2:** extraction column;
**B1:** condensate tank;
**E1-E4:** heat exchanger;
**1-8:** pipeline No. or material stream No.;
**R:** reaction zone;
**T:** debutanizer area;
**T1:** reactive distillation zone;
**C:** distillation-extraction coupling zone.

### DETAILED DESCRIPTION OF THE EMBODIMENTS

The present invention is explained in further detail below with reference to the attached drawing 1 and embodiment. A process of coupling separation and purification to produce ETBE, in accordance with the invention, comprises the following steps:

The make-up ethanol containing higher than 1 wt% up to 15 wt% of water and/or the recovered ethanol or the mixture thereof as well as the mixture mainly containing ETBE and ethanol are mixed via pipeline 2 and 1 respectively, and then introduced into the distillation column **C1** via pipeline **3** and the heat exchanger **E1** to undergo heat exchange. The top of the distillation column **C1** is disposed with the condenser **E2** and the condensate tank **B1,** and the bottom of the distillation column **C1** is disposed with the heater **E3.** Pipeline **4** is used to collect the fraction containing ethanol at the bottom of the distillation column **C1,** and the material obtained at the top of the distillation column **C1** is fed into the extraction column **C2** via pipeline **5** and the heat exchanger **E4** to conduct heat exchange.

Water, as the extraction agent, is introduced via pipeline 6 into the extraction column **C2** to conduct extraction, the extract liquor containing ethanol is collected via pipeline 7 at the bottom of the extraction column **C2,** and the extract liquor can be recycled after the recovery of ethanol ( many existing methods to recover ethanol are available, such as the enrichment recovery in a recovery tower which is not shown in the drawing, the recovered ethanol can be reintroduced to the distillation column **C1,** and the recovered, water with ethanol having been removed can be recycled into the extraction column **C2** and excessive water can be discharged), and the extraction raffinate containing ETBE is collected via pipeline **8** at the top of the extraction column **C2.**

The content of ETBE, ethanol and water in the feeding material of the distillation column **C1** can be allocated to some extent so as to allow almost all water, under the operating conditions of the distillation column **C1,** to be taken out of the top of the distillation column **C1** in the form of a ternary azeotropy. Even a small amount of remaining ETBE can be removed from the top of the distillation column through a binary azeotropy of ETBE/water and/or ETBE/ethanol. For example, under 1 Pa of the operating pressure, the referential composition of the ternary azeotropy contains approximately 82.9 wt% of ETBE, 11.6 wt% of ethanol and 5.5 wt% of water. The binary azeotropy of ETBE/water contains 6 wt% of water and the binary azeotropy of ETBE/ethanol contains 21 wt% of ethanol (refer to Lee H. Horsley: Azeotropic Data—III. American Chemical Society /Washington, D.C. 1973, US 5250156A, Pure Appl. Chem., Vol. 71, No. 6, pp. 939-949, 1999 for the azeotrope data). In fact, as ethanol is often excessive, it is common to control the intake of ethanol according to its water content so as to obtain the ethanol containing acceptable amount of water at the bottom of the distillation column **C1.**

The preferred weight ratio (i.e. water/ETEB weight ratio) between the total water content of said make-up ethanol and/or the recovered ethanol or the mixture thereof, respectively or collectively introduced to the distillation column **C1,** and the amount of ETBE in the mixture mainly containing ETBE and ethanol, respectively or collectively introduced to the distillation column **C1,** is 0.008-0.18:1, preferably 0.014-0.12:1 and most preferably 0.02-0.09:1. Or, the preferred weight ratio (i.e. water/ETEB weight ratio) between the total water content of the said make-up ethanol and/or the recovered ethanol or the mixture thereof and the mixture mainly containing ETBE and ethanol, respectively or collectively introduced to the distillation column **C1,** and the amount of ETBE in the mixture mainly containing ETBE and ethanol, respectively or collectively introduced to the distillation column **C1,** is 0.008-0.18:1, more preferably 0.014-0.12:1 and most preferably 0.02-0.09:1.

The operating pressure of the distillation column **C1** is 0.5-2 Pa (preferably 1-2 Pa). The temperature at the top of the distillation column **C1** is 45-80°C, and the temperature at the bottom of the distillation column **C1** is 60-95°C. The preferred feed temperature is 10-80°C, more preferably 50-80°C and most preferably at the temperature close to the ternary azeotropic point of ETBE/ethanol/water under the operating pressure of the distillation column **C1.**

The operating pressure of the extraction column **C2** is 1-15 Pa (preferably 1-10 Pa). The operating temperature of the extraction column **C2** is 5-70°C (preferably 10-60°C). The preferred weight ratio between the consumption of the water, as the extraction agent, and the extraction feed is normally no more than 1.2:1, more preferably 0.2-1.0:1 and most preferably 0.3-0.8:1.

The ethanol content in the extraction raffinate containing ETBE collected at the top of the extraction column **C2** is related to the operating conditions and generally it is less than 1 wt%.

The extraction raffinate containing ETBE collected at the top of the extraction column **C2** is ETBE containing saturated water. Only a small amount of water is left after ethanol is removed under normal temperature. For example, the saturated water content of ETBE under the temperature of 20°C is 0.5 g/100 g of ETBE and generally it is not necessary to further remove the water. However, if it is necessary, appropriate water removal methods can be applied, for example, using absorbents such as molecular sieve or silica gel to remove the water. Alternatively, if the material is obtained from the side of the debutanizer for coupling separation and purification, it can be fed into the debutanizer again, and the ETBE containing almost totally no water can be obtained at the bottom of the debutanizer.

The water content of the fraction containing ethanol collected at the bottom of the distillation column **C1** is related to the operating conditions and generally it is less than 1 wt%., or even less than 0.5 wt%. The ethanol content of the fraction containing ethanol collected at the bottom of the distillation column **C1** is related to the operating conditions and preferably it is more than 99 wt% and more preferably even more than 99 mol%.

It can be useful that the ratio of molar flow rate or mole number of the ethanol in the said make-up ethanol, respectively or collectively introduced into the distillation column **C1,** to the ETBE in the mixture mainly containing ETBE and ethanol, respectively or collectively introduced into the distillation column **C1,** is 1-25.9:1, appropriately 1-12.8:1, more appropriately 1-6.3:1 and the most appropriately 1-5:1.

The condensate and/or reflux condensed at the top of the distillation column **C1** may generally contain a small amount of aqueous phase according to different operating conditions. All or part of the aqueous phase (aqueous solution containing ethanol) therein can be removed by allowing at least part of the condensate and/or reflux to undergo oil-water separation. After that, at least part of the condensate and/or reflux can be used as the reflux for the distillation column **C1** and/or the recycled feed for the distillation column **C1.** It is preferred to carry out the oil-water separation of at least the part of the condensate and/or reflux at the temperature of 10-60°C and more preferably at the temperature of 10-40°C. The aqueous phase (aqueous solution containing ethanol) separated out of oil-water separation can be further treated (not shown in the drawing) and recycled after the ethanol has been recoverd, or sent to extraction treatment along with the rest of the condensate containing at least part of oil phase and/or aqueous phase.

In such a way, it is also possible to apply the process of coupling separation and purification to produce ETBE provided by the invention to the make-up ethanol containing at least higher than 1 wt% up to 15 wt% of water (preferably the make-up ethanol contains 1.5-15 wt% of water and more preferably 2-10 wt% of water, and the most preferably 3-10 wt% of water) to produce ethanol, that means, the process can be used to remove water from the ethanol by means of coupling separation and purification. The ethanol content of thus obtained fraction containing ethanol is related to the operating conditions, wherein the preferred ethanol content is more than 99 wt% and more preferably even more than 99 mol%.

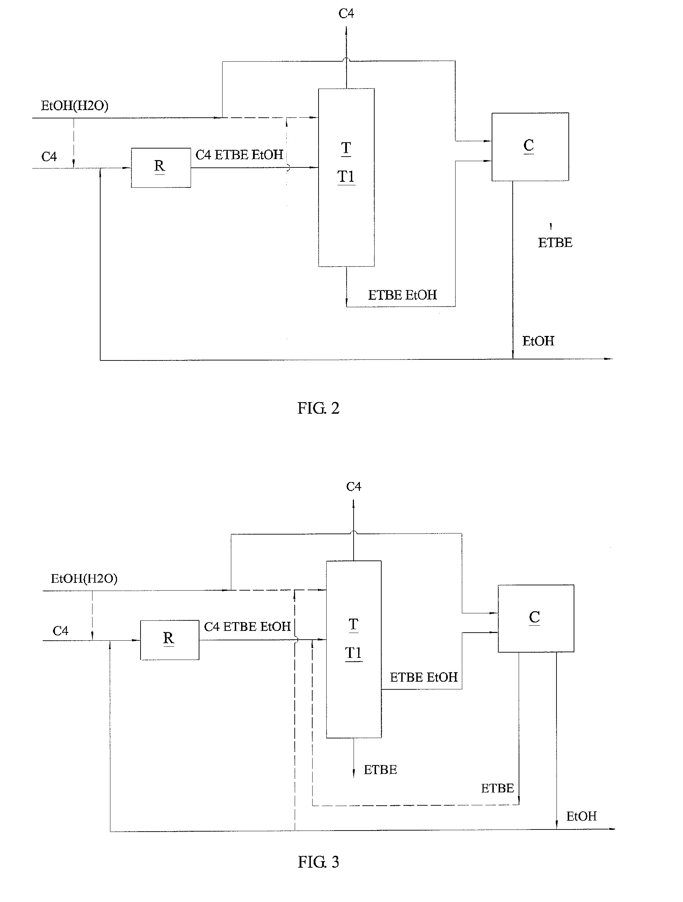

As shown in FIG. **2****,** a process of coupling separation and purification to produce ETBE (including the process to take off the material from the bottom of the debutanizer area), in accordance with the invention, comprises the following step:

Allow ethanol and mixed C₄-hydrocarbons containing isobutene (e.g. from steam cracking or catalytic cracking) to undertake etherification at the reaction zone **R** (may includes one or more reactors), referring to US 5248836A, CN 1990443A, CN1772848A and CN101195560A for reaction conditions.

The reaction products obtained from the reaction zone **R** mainly comprise ETBE, ethanol and unreacted C₄-hydrocarbons. Separate the reaction products in a debutanizer area **T** (i.e. normally so-called debutanizer), collect the fraction containing hydrocarbons at the top of the debutanizer and collect the mixture mainly containing ETBE and ethanol at the bottom of the debutanizer. If it is necessary to further increase the conversion of isobutene, a reactive distillation zone **T1** can be used instead. As shown in FIG. **2** (dotted line), the make-up ethanol can be added from the top of the reactive distillation zone **T1.**

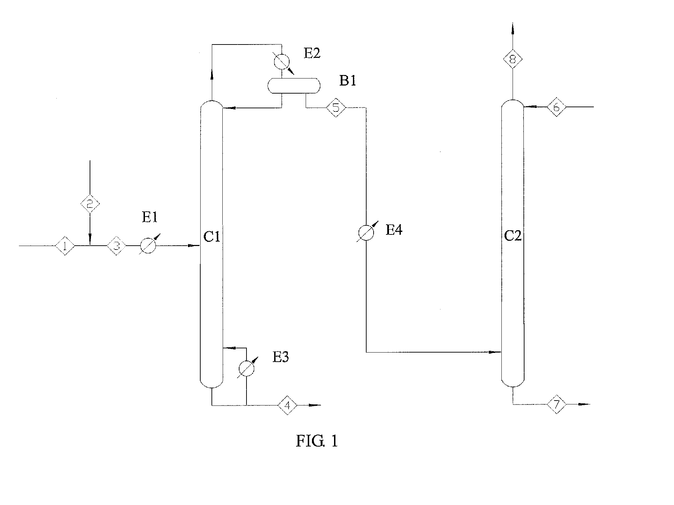

The mixture mainly containing ETBE and ethanol collected at the bottom of the debutanizer area **T** or the reactive distillation zone **T1,** the ethanol containing higher than 1 wt% up to 15 wt% of water or preferably further the make-up ethanol containing higher than 1 wt% up to 15 wt% of water and/or the recovered ethanol or the mixture thereof are respectively or collectively introduced to a distillation-extraction coupling zone **C** (it is composed of the distillation column **C1** and the extraction column **C2)** and then the process of coupling separation and purification to produce ETBE provided by the invention is used to operate (as shown in FIG. **1****).** Thus, the extraction raffinate containing ETBE can be collected at the top of the extraction column **C2,** the extraction liquor containing ethanol can be collected at the bottom of the extraction column **C2,** and the fraction containing ethanol can be collected at the bottom of the distillation column **C1.**

Generally, the hydrocarbons collected at the top of the debutanizer area **T** or the reactive distillation zone **T1** further contain a small amount of azeotropic ethanol. Therefore, the ethanol must be removed by water scrubbing for the conventional process to produce tert-alkyl ether and a recovery column has to be arranged to recover the ethanol (see US 5447607A for detail). As a result, the recovery column can be used to recover the ethanol from the extract liquor containing ethanol collected from the distillation-extraction coupling zone C in the same way, and followed by recycling the extract liquor into the distillation-extraction coupling zone C (not shown in the drawing).

A probably more useful process is that firstly, the said mixture mainly containing ETBE and ethanol, the said make-up ethanol, the said recovered ethanol or the mixture thereof are fed into the distillation-extraction coupling zone **C** for coupling separation and purification, and then the fraction containing ethanol is collected at the bottom of the distillation column **C1,** after that at least part of the fraction is used as the feeding material for the reaction zone **R** or/and the reactive distillation zone **T1.** In this way, when the device is started it probably needs or only needs to firstly provide the reaction zone **R** with the start-up ethanol from which azeotropic water has been substantially removed.

A possible preferred process is that all ethanol feed for the reaction zone **R** and/or the reactive distillation zone **T1** is from the ethanol in which azeotropic water has been substantially removed obtained from the distillation-extraction coupling zone C (i.e. the fraction containing ethanol collected at the bottom of the distillation column **C1).**

As shown in FIG. 3, a process of coupling separation and purification to produce ETBE (including the process to take off materials from the side of the debutanizer area), in accordance with the invention, comprises the following step:

Allow ethanol and mixed C₄-hydrocarbons containing isobutene (e.g. from steam cracking or catalytic cracking) to undertake etherification at the reaction zone **R** (may includes one or more reactors), referring to US 5248836A, CN 1990443A, CN 1772848A and CN101195560A for reaction conditions.

The reaction products obtained from the reaction zone R mainly include ETBE, ethanol and unreacted C₄-hydrocarbons. The reaction products are separated in the debutanizer area **T** (i.e. normally so-called debutanizer), the fraction containing hydrocarbons is collected at the top of the debutanizer and the fraction containing ETBE is collected at the bottom of the debutanizer. If it is necessary to further increase the conversion of isobutene, a reactive distillation zone **T1** can be used instead. At this time, as shown in FIG. **3** (dotted line), make-up ethanol can be added from the top of the reactive distillation zone **T1.**

The mixture mainly containing ETBE and ethanol taken off the side of the debutanizer area **T** or the reactive distillation zone **T1,** said side take-off means that the take-off point is located at the side position below the feed inlet point of the reaction products obtained from the reaction zone R, more preferably at the side position in which the tray shows substantially the highest ethanol concentration or the side position below the tray, and most preferably at the side position in which the tray shows substantially the highest ethanol concentration or the side position in which the tray is located between the 1st-15th theoretical plates below the tray with substantially the highest ethanol concentration (including the first and the fifteenth theoretical plates); the mixture mainly containing ETBE and ethanol is taken off in vapor phase and/or liquid phase at the side of the debutanizer area **T** or the reactive distillation zone **T1.**

Or referring to CN1127243A and US 5,607,557A for the side take-off method, the ethanol containing higher than 1 wt% up to 15 wt% of water or preferably the make-up ethanol containing higher than 1 wt% up to 15 wt% of water and/or the recovered ethanol or the mixture thereof are further respectively or collectively introduced to a distillation-extraction coupling zone **C** (said distillation-extraction coupling zone is composed of the distillation column **C1** and the extraction column **C2)** and then apply the process of coupling separation and purification to produce ETBE provided by the invention (as shown in FIG. 1). Consequently, the extraction raffinate containing ETBE can be collected at the top of the extraction column **C2,** the extraction liquor containing ethanol can be collected at the bottom of the extraction column **C2** and the fraction containing ethanol can be collected at the bottom of the distillation column **C1** as well.

It is probably more useful that 0-100% of the collected extraction raffinate containing ETBE is circulated to the debutanizer area **T** or the reactive distillation zone **T1,** as shown in FIG. **3** (dotted line), in such a way, the fraction containing ETBE collected at the bottom of the debutanizer area **T** or the reactive distillation zone **T1** not only contains less than 1 wt% of ethanol but almost no water.

Generally, the hydrocarbons collected at the top of the debutanizer area **T** or the reactive distillation zone **T1** further contain a small amount of azeotropic ethanol, therefore, the ethanol must be removed by water scrubbing for the conventional process to produce tert-alkyl ether and a recovery column has to be arranged to recover the ethanol (see US 5447607A for detail). As a result, the recovery column can be used to recover the ethanol from the extract liquor containing ethanol collected from the distillation-extraction coupling zone **C** in the same way, and followed by recycling it into the distillation-extraction coupling zone **C** (not shown in the drawing).

A possibly useful process is that firstly, said mixture mainly containing ETBE and ethanol, then said make-up ethanol and the recovered ethanol or the mixture thereof are fed into the distillation-extraction coupling zone **C** for coupling separation and purification, and then the fraction containing ethanol is collected at the bottom of the distillation column **C1,** after that at least part of the fraction can be used as the feeding material for the reaction zone R or/and the reactive distillation zone **T1.** In this way, when the device is started up it probably needs or needs only to firstly provide the reaction zone **R** with the start-up ethanol from which azeotropic water has been substantially removed.

A possible preferred process is that all the ethanol feed into the reaction zone R and/or the reactive distillation zone **T1** comes from the ethanol in which azeotropic water has been substantially removed obtained from the distillation-extraction coupling zone **C** (i.e. the fraction containing ethanol collected at the bottom of the distillation column **C1).**

The present invention is explained in further detail below with reference to the examples.

Conduct coupling separation and purification to the mixture mainly containing ETBE and ethanol (the mixture is obtained from the side or at the bottom of the debutanizer behind the reaction zone R used to produce said ETBE), the ethanol containing higher than 1 wt% up to 15 wt% of water or the make-up ethanol containing higher than 1 wt% up to 15 wt% of water and/or the recovered ethanol or the mixture thereof so as to obtain the qualified ETBE product (the ethanol content is less than 1 wt%) at the top of the extraction column **C2** and the ethanol in which azeotropic water has been substantially removed (water content is less than 1 wt% or even less than 0.5 wt%) obtained at the bottom of the distillation column **C1.**

### Example 1

The mixture of ETBE and ethanol to be separated contains 80 wt% of ETBE and 20 wt% of ethanol and ethanol feed containing azeotropic water contains 94.6 wt% of ethanol and 5.4 wt% of water.

Distillation column **(C1)** is a glass tower composed of 70 pieces of perforated tray with tray weir, with the tray spacing of 50mm and the inner diameter of 50mm. The operating pressure of the distillation column **C1** is 0.5-2 Pa (preferably 1-2 Pa). The temperature at the top of the distillation column **C1** is 45-80°C and the temperature at the bottom of the distillation column **C1** is 60-95°C. The preferred feeding temperature is 10-80°C, more preferably 50-80°C, and most preferably close to the temperature at the ternary azeotropic point of ETBE/ethanol/water under the operating pressure of the distillation column **C1.**

Extraction column **(C2)** is a stainless tower composed of 24 pieces of perforated tray with the tray spacing of 100mm and the inner diameter of 30mm. The operating pressure of the extraction column **C2** is 1-15 Pa (preferably 1-10 Pa). The operating temperature of the extraction column **C2** is 5-70°C (preferably 10-60°C). The preferred weight ratio between the water (as the extraction agent) consumption and the extraction feed is no more than 1.2:1, more preferably 0.2-1.0:1 and most preferably 0.3-0.8:1.

The distillation column **C1** is thermally insulated to prevent heat loss. The trays of both towers (e.g. distillation column **C1** and the extraction column **C2)** are numbered from top to bottom.

The mixture of ETBE and ethanol to be separated passes through pipeline 1 and ethanol containing azeotropic water passes through pipeline **2,** after they are mixed via pipeline **3** and heat exchanged to 65°C via heat exchanger **E1,** they are fed into the distillation column **C1** on the 20th tray. The operating pressure of the distillation column **C1** is 1 Pa, the temperature at the top of the distillation column **C1** is 64°C and the temperature at the bottom thereof is 79°C. The reflux ratio is set to 4:1 (the temperature is approximately 30°C for the condensate, which contains nearly 2 wt% of aqueous phase). The fraction containing ethanol, whose water content is even less than 0.3 wt%, is collected via pipeline **4** at the bottom of the column. The effluent from the top of the column is fed into the extraction column **C2** on the 24th tray after passing through pipeline **5** and heat exchanging to 30°C by heat exchanger **E1.** Feed water of 20°C into the extraction column **C2** on the first tray via pipeline **6,** and the operating pressure of the extraction column **C2** is 1 Pa. Consequently, the extraction raffinate containing ETBE is collected at the top of the extraction column **C2** via pipeline **8,** and the extract liquor containing ethanol is collected at the bottom of the extraction column **C2** via pipeline **7** while the ethanol can be further recovered to be recycled. Table 1 shows the material balance for each material streams passing through each pipeline, including the mixture of ETBE and ethanol to be separated and ethanol containing azeotropic water.

**Table 1 Material Balance Table of Example 1**

| Composition\wt%\pipelines | 1 | 2 | 3 | 4 | 5 | 6 | 7 | 8 |
|---|---|---|---|---|---|---|---|---|
| H₂O | | 5.4 | 2.3 | 0.2 | 4.0 | 100 | 78.2 | 0.8 |
| EtOH | 20 | 94.6 | 52.0 | 99.8 | 12.9 | | 20.1 | 0.1 |
| ETBE | 80 | | 45.7 | <100ppm | 83.1 | | 1.7 | 99.1 |
| Flow rate, kg/h | 1.20 | 0.90 | 2.10 | 0.95 | 1.16 | 0.54 | 0.74 | 0.96 |

### Example 2

The mixture of ETBE and ethanol to be separated contains 80 wt% of ETBE and 20 wt% of ethanol. The mixture feed of the make-up ethanol containing azeotropic water and the recovered ethanol contains 93.4 wt% of ethanol, 5.3 wt% of water and 1.3 wt% of ETBE.

The devices, operating process and conditions in example 2 are the same as those described in example 1. Table 2 shows the material balance for each material streams passing through each pipeline, including the mixture of ETBE and ethanol to be separated and the mixture of the make-up ethanol containing azeotropic water and the recovered ethanol.

**Table 2 Material Balance Table of Example 2**

| Composition\wt%\pipeline | 1 | 2 | 3 | 4 | 5 | 6 | 7 | 8 |
|---|---|---|---|---|---|---|---|---|
| H₂O | | 5.3 | 2.3 | 0.2 | 4.0 | 100 | 78.2 | 0.8 |
| EtOH | 20 | 93.4 | 51.7 | 99.8 | 13.0 | | 20.1 | 0.1 |
| ETBE | 80 | 1.3 | 46.0 | <100ppm | 83.0 | | 1.7 | 99.1 |
| Flow rate, kg/h | 1.20 | 0.91 | 2.11 | 0.94 | 1.17 | 0.54 | 0.74 | 0.97 |

In accordance with the process and device of purification to produce ETBE of the invention, the purification process to produce ETBE is carried out by coupling separation. The ETBE obtained by means of purification may contain no more than 1 wt% of ethanol and the obtained ethanol may contain less than 1 wt% of water. According to the process and device provided by the invention, there are no strict requirements (including ethanol content and/or water content and/or tertiary alcohol content) on the mixture (obtained at the bottom or side of the debutanizer behind the reaction zone) mainly containing ETBE and ethanol. The renewable ethanol containing at least higher than 1 wt% up to 15 wt% of water and the mixture to be separated mainly containing ETBE and ethanol that are hard to be directly separated can be coupled together for coupling separation in the distillation-extraction coupling zone to achieve multiple coupling such as cogeneration coupling of ETBE and ethanol products. Meanwhile, ethanol feed can be provided for etherification and/or ethanol products provided as gasoline additive as required. The ethanol/olefine ratio can be appropriately increased or widened for the etherification so as to increase the conversion of isobutene and/or selectivity of the etherification within a certain range. Therefore, the invention is possessed of high commercial values (i.e. functions/costs).

## Claims

1. A process of coupling separation and purification to produce ETBE, comprising:
Feeding ethanol which contains higher than 1 wt% but up to 15 wt% of water and a mixture containing ETBE and ethanol into a distillation-extraction coupling zone for the coupling separation and purification, wherein said distillation-extraction coupling zone is composed of a distillation column and an extraction column, and the fraction containing ETBE, ethanol and water is collected at the top of the distillation column while the fraction containing ethanol is collected at the bottom of the distillation column; then
feeding the fraction containing ETBE, ethanol and water collected at the top of the distillation column into the extraction column therein said fraction being extracted by water as the extraction agent, and collecting the extract liquor containing ethanol at the bottom of the extraction column and the extraction raffinate containing ETBE at the top of the extraction column.

2. The process of claim 1, wherein the make-up ethanol containing higher than 1 wt% up to 15 wt% of water and/or the recovered ethanol or the mixture thereof as well as the mixture containing ETBE and ethanol are fed into the distillation-extraction coupling zone for coupling separation and purification.

3. The process of claim 1 or 2, wherein said mixture containing ETBE and ethanol contains 5-50 wt% of ethanol based on the total weight of ETBE and ethanol contained in said mixture thereof.

4. The process of claim 2, wherein the make-up ethanol contains 1.5-15 wt% of water; or the weight ratio between the total water content of the make-up ethanol and/or the recovered ethanol or the mixture thereof and the ETBE in the mixture containing ETBE and ethanol is 0.008-0.18:1; or the weight ratio between the total water content of the make-up ethanol and/or the recovered ethanol or the mixture thereof as well as the mixture containing ETBE and ethanol, and the ETBE in the mixture containing ETBE and ethanol is 0.008-0.18:1; or the ratio of molar flow rate or mole number between the ethanol in the make-up ethanol and the ETBE in the mixture containing ETBE and ethanol, respectively or collectively fed into the distillation column, is 1-25.9:1.

5. The process of claim **2,** wherein the make-up ethanol contains 3-10 wt% of water; or the weight ratio between the total water content of the make-up ethanol and/or the recovered ethanol or the mixture thereof and the ETBE in the mixture containing ETBE and ethanol is 0.02-0.09:1; or the weight ratio between the total water content of the make-up ethanol and/or the recovered ethanol or the mixture thereof as well as the mixture containing ETBE and ethanol, and the ETBE in the mixture containing ETBE and ethanol is 0.02-0.09:1; or the ratio of molar flow rate or mole number between the ethanol in the make-up ethanol and the ETBE in the mixture containing ETBE and ethanol, respectively or collectively fed into the distillation column, is 1-6.3:1.

6. The process of any of claims **1, 2, 4** and **5,** wherein the extraction raffinate containing ETBE collected at the top of the extraction column contains less than 1 wt% of ethanol and/or the fraction containing ethanol collected at the bottom of the distillation column contains less than 1 wt% of water.

7. A process of coupling separation and purification to produce ETBE, comprising:
the make-up ethanol and/or the recovered ethanol or the mixture thereof ethanol containing higher than 1 wt% up to 15 wt% of water, and a mixture containing ETBE and ethanol are fed into a distillation-extraction coupling zone for separation and purification, wherein said distillation-extraction coupling zone is composed of a distillation column and an extraction column, the fraction containing ETBE, ethanol and water is collected at the top of the distillation column while the fraction containing ethanol is collected at the bottom of the distillation column; and
at least part of the condensate and/or the reflux flowing out of the top of the distillation column can be used as the reflux for the distillation column and/or recycled as the feed for the distillation column after aqueous phase therein is removed by means of oil-water separation; the rest of the fraction containing ETBE, ethanol and water is then fed to the extraction column and extracted by water as the extraction agent; the extract liquor containing ethanol is collected at the bottom of the extraction column while the extraction raffinate containing ETBE is collected at the top of the extraction column.

8. The process of claim **7,** wherein the operating temperature for oil-water separation of at least part of the condensate and/or reflux flowing out of the top of the distillation column is 10-40°C.

9. The process of any of claims **1, 2, 4, 5, 7** and **8,** wherein the ethanol contained in the extract liquor collected at the bottom of the extraction column is recovered to be recycled as the feed of the distillation column.

10. A process of coupling separation and purification to produce ETBE, comprising:
**a.** feeding ethanol and mixed C₄-hydrocarbons containing isobutylene into a reaction zone **(R)** for etherification ;
**b.** feeding the material containing ETBE, ethanol and C₄-hydrocarbons flowing out of the reaction zone **(R)** into a debutanizer area **(T)** or a reactive distillation zone **(T1)** for debutanizer distillation or reactive distillation, and then collecting fraction containing C₄-hydrocarbons at the top of the reactive distillation zone **(T1)** and taking off the mixture containing ETBE and ethanol from the bottom or side thereof;
**c.** feeding the mixture containing ETBE and ethanol taken off the bottom or side of the debutanizer area **(T)** or the reactive distillation zone **(T1)** and the ethanol containing higher than 1 wt% up to 15 wt% of water into the distillation-extraction coupling zone **(C)** for coupling separation and purification in accordance with the process described in any of claims 1-9, and then collecting the fraction containing ethanol at the bottom of the distillation column **(C1),** collecting the extraction raffmate containing ETBE at the top of the extraction column **(C2)** and collecting the extract liquor containing ethanol at the bottom of the extraction column **(C2)** in the distillation-extraction coupling zone **(C).**

11. The process of claim **10,** wherein when the mixture containing ETBE and ethanol is taken off the side of the debutanizer area **(T)** or the reactive distillation zone **(T1),** 0-100% of the extraction raffinate containing ETBE collected at the top of the extraction column **(C2)** in the distillation-extraction coupling zone **(C)** is recycled into the debutanizer area **(T)** or the reactive distillation zone **(T1)** so as to collect the fraction containing ETBE at the bottom of the debutanizer area **(T)** or the reactive distillation zone **(T1).**

12. The process of claim **11,** wherein the position to take off the mixture containing ETBE and ethanol from the side of the debutanizer area **(T)** or the reactive distillation zone **(T1)** is located at the side position in which the tray shows substantially the highest ethanol concentration or the side position below the tray, said mixture containing ETBE and ethanol is taken off the vapor phase and/or liquid phase at the side of the debutanizer area **(T)** or the reactive distillation zone **(T1).**

13. The process of claim **12,** wherein the position to take off the mixture containing ETBE and ethanol from the side of the debutanizer area **(T)** or the reactive distillation zone **(T1)** is located at the side position in which the tray shows substantially highest ethanol concentration or located between the 1st-15th theoretical tray below the tray with substantially the highest ethanol concentration.

14. The process of any of claims **10-13,** wherein the ethanol feed for the reaction zone **(R)** and/or the reactive distillation zone **(T1)** comes from the fraction containing ethanol collected at the bottom of the distillation column **(C1)** in the distillation-extraction coupling zone **(C).**

15. A device of coupling separation and purification to produce ETBE, comprising a distillation column **(C1)** and an extraction column **(C2),** wherein
the distillation column **(C1)** is disposed with pipeline **(3)** for introducing distillation feed, the bottom of the column is disposed with a heater **(E3)** and pipeline **(4)** to collect the fraction containing ethanol, and the top of the column is disposed with a condenser **(E2)** and a condensate tank **(B1)** to collect the fraction containing ETBE, ethanol and water as well as pipeline **(5)** to feed the material obtained at the top of the distillation column **(C1)** into the extraction column **(C2);** it further comprises:
the distillation column is also disposed with pipeline **(6)** to feed the extraction agent into the extraction column **(C2),** pipeline **(7)** to collect the extract liquor containing ethanol at the bottom of the extraction column **(C2),** and pipeline **(8)** to collect the extraction raffinate containing ETBE at the top of the extraction column **(C2).**
